Europäisches Patentamt

European Patent Office

Office européen des brevets

(11) Publication number: **0 200 383**
**A2**

## (12) EUROPEAN PATENT APPLICATION

(21) Application number: 86302537.5

(22) Date of filing: 07.04.86

(51) Int. Cl.⁴: **A61K 37/26** , **A61K 47/00** , **A61K 9/72**

(30) Priority: 15.04.85 US 723231

(43) Date of publication of application:
10.12.86 Bulletin 86/45

(84) Designated Contracting States:
BE CH DE FR GB IT LI NL SE

(71) Applicant: **ELI LILLY AND COMPANY**
**Lilly Corporate Center**
**Indianapolis Indiana 46285(US)**

(72) Inventor: **Su, Kenneth Shyan-Ell**
**8530 Trails Run Road**
**Indianapolis Indiana 46217(US)**
Inventor: **Campanale, Kristina Marie**
**14461 Allisonville Road**
**Noblesville Indiana 46060(US)**

(74) Representative: **Tapping, Kenneth George et al**
**Erl Wood Manor**
**Windlesham Surrey, GU20 6PH(GB)**

(54) **An improved method for administering insulin.**

(57) A treatment of diabetes mellitus is described. The method comprises administering, via the nasal mucous membrane of a patient having diabetes mellitus, a pharmaceutically acceptable amount of an alkali metal salt, the ammonium salt, or the free acid of a substantially zinc-free insulin, optionally in the presence of an absorption enhancing agent.

FIG.I COMPARISON OF BLOOD GLUCOSE AFTER INTRANASAL ADMINISTRATION OF PORK Zn INSULIN AND PORK Na INSULIN AT THE DOSE OF 4 U/kg IN RATS

EP 0 200 383 A2

# AN IMPROVED METHOD FOR ADMINISTERING INSULIN

For many years nasal absorption has been viewed as a potentially attractive route for administering polypeptides. However, major obstacles limiting sustained interest in intranasal administration have centered on the difficulty (a) of achieving sufficiently high levels of absorption and (b) precisely and consistently controlling the amount of polypeptide received.

In particular, these difficulties have been apparent in attempting to administer insulin intranasally. Therefore, it has been accepted generally that treatment of diabetic patients with insulin can be achieved effectively only by parenteral injection. Nevertheless, local discomfort and perceived disruption of normal lifestyle deter many diabetics from readily accepting the present means of insulin treatment. For these reasons, other routes of insulin administration continue to be sought.

Over recent years a renewed interest in intranasal administration of insulin has occurred. In part, this renewed interest is founded upon the development of nebulizers which consistently deliver a controlled dose of the desired entity to the nasal passage. Consistent delivery to the nasal mucosa of precisely controlled amounts of insulin thus can be a reality, and practical intranasal administration of insulin now is largely dependent upon achieving sufficiently high levels of intranasal insulin absorption.

The literature to date has suggested that uptake of insulin from the nasal mucosa is possible only by incorporation into the formulation of additional agents. Thus, Hirai et al., U.S. Patent No. 4,153,689, describe stable aqueous insulin solutions useful for intranasal administration which have a pH value in the range of from 2.5 to 4.7 and which contain from 0.1 to 20 weight percent of a stabilizing agent selected from the group consisting of (a) at least one non-ionic surface active agent whose hydrophile-lipophile balance value is in the range of from 9 to 22, (b) polyethylene glycol having a molecular weight in the range of from 200 to 7500 and (c) mixtures of (a) and (b).

Hirai, S., Yashiki, T., and Mima, H., Internatl. J. of Pharm. 9, 165-172 (1981) describe enhancement of insulin absorption through the nasal mucosa by addition to the insulin solution of a surfactant, for example, sodium glycocholate, saponin, or polyoxyethylene-9-lauryl ether [see, in addition, Hirai, S., Yashiki, T., and Matsuzawa, T., Diabetes 27, 296-299 (1978)].

Recently, Moses, A.C., Gordon, G.S., Carey, M.C., and Flier, J.S., Diabetes 32, 1040-1047 - (1983), reported data that showed intranasal insulin absorption in humans was achieved by concomitant administration of bile salt.

All studies reported to date have focused upon the incorporation in an insulin solution of an adjuvant, generally an absorption promoter, that is, a surfactant such as a non-ionic surface active agent; a bile salt, or a chelating agent.

Achieving high levels of insulin absorption intranasally now is possible by using insulin alone when such insulin is in the form of an alkali metal or ammonium salt, or is a free acid. This invention is directed to this means for treating diabetes mellitus.

Furthermore, in the context of the specific insulins used in this invention, further enhancement of intranasal absorption is possible by incorporating with such insulins, either alone or jointly, selected absorption enhancing agents.

In accordance with the invention, there is provided a means for the treatment of diabetes mellitus which comprises administering to a warm-blooded animal, via the nasal mucous membrane of said animal having diabetes mellitus a pharmaceutically acceptable amount of an alkali metal salt, the ammonium salt, or the free acid of a substantially zinc-free insulin.

In particular, in one embodiment, there is provided an intranasal pharmaceutical formulation containing insulin for the treatment of diabetes mellitus characterized in that said intranasal formulation is comprised of insulin as an alkali metal salt, the ammonium salt or the free acid of a substantially zinc-free insulin, and a pharmaceutically acceptable diluent therefor.

In an additional embodiment this invention is directed to an intranasal pharmaceutical formulation comprising a pharmaceutically acceptable amount of an alkali metal salt, the ammonium salt, or the free acid of a substantially zinc-free insulin, and an absorption enhancing amount of at least one absorption enhancing agent selected from the group consisting of (1) oleic acid or an ester or salt thereof, (2) a liquid form sorbitan fatty acid ester, - (3) a liquid form polyoxyethylene derivative of a sorbitan fatty acid ester, and (4) a liquid form hydroxypolyoxyethylene-polyoxypropylene-polyoxyethylene copolymer.

Figure 1 is a plot comparing the blood glucose level following intranasal administration of porcine zinc insulin and porcine sodium insulin at a dose of 4 Units per kilogram in rats.

Figure 2 is a plot comparing the blood glucose level following intranasal administration of human zinc insulin and human sodium insulin at a dose of 5 Units per kilogram in rats.

Figure 3 is a plot comparing the blood glucose level following intranasal administration of human metal-free insulin and human sodium insulin at a dose of 5 Units per kilogram in rats.

As noted, this invention relates to the use of a metal-free insulin or the ammonium or an alkali metal salt of an insulin. "Alkali metal salt" means, for example, the sodium salt, the potassium salt, the lithium salt, and the like. Of the foregoing metal-free insulins and insulin salts, the sodium salt is highly preferred for use in the method of this invention.

The insulins used in the method of this invention also are substantially zinc-free. "Substantially zinc-free" means an insulin to which zinc has not been added.

The insulins used in the method of this invention can have the structure of any of a wide range of species, in particular, bovine, porcine, or human, and, most particularly, human.

The insulin administered intranasally may be administered as a composition. Compositions suitable for such intranasal administration conveniently comprise sterile aqueous solutions or sterile suspensions of the pharmaceutically active ingredient. The sterile aqueous solution can be isotonic with the blood of the recipient, generally using sodium chloride, glycerin, glucose, mannitol, sorbitol, and the like. In addition, the compositions, whether solution or suspension, may contain any of a number of adjuvants, such as buffers, preservatives, stabilizing agents, agents that promote rapid onset of action, agents that promote prolonged duration of action, and the like. Typical preservatives are, for example, phenol, $\underline{m}$-cresol, methyl $\underline{p}$-hydroxybenzoate, propyl $\underline{p}$-hydroxybenzoate, 1,1,1-trichloro-2-methyl-2-propanol (chlorbutol), benzalkonium chloride, ethylenediaminetetraacetic acid (EDTA), N,N-dimethyl-N-(2-phenoxyethyl)-1-dodecanaminium bromide (domiphen bromide), and the like. Although a buffer may be present in the compositions used, preferably the composition is free of any buffering agent. When present, however, typical buffers are, for example, sodium phosphate, sodium acetate, sodium citrate, and the like.

One or more emulsifying agents may be added to the composition used in the method of this invention in order to improve its stability. The emulsifier employed may be chosen from among those generally known to be suitable for use in pharmaceutical formulations. Typical emulsifiers may be natural emulsifying agents, such as lecithin, and the like. Synthetic emulsifying agents can also be used. Depending upon the charge possessed by the emulsifier, the class may be divided into anionic, cationic, and nonionic types. The preferred emulsifier will be nonionic, and, in particular, a fatty acid, sorbitan fatty acid ester, or a polyoxyethylene derivative thereof. Examples of such emulsifying agents are, for example, oleic acid, linoleic acid, linolenic acid, ethyl oleate, methyl linoleate, methyl linoleate, and the like. A sorbitan fatty acid ester emulsifier is available commercially under the trademark Span. A polyoxyethylene type of emulsifier is commercially available under the trademark Tween. A highly preferred emulsifier is Tween 85, which is polyoxyethylene 20 sorbitan trioleate, and is available from ICI Americas. Other suitable emulsifiers include the poloxamers including the Pluronic emulsifiers from BASF Wyandotte such as Pluronic L81. Blends of emulsifiers are common and may be used also. The reader is referred to REMINGTON PHARMACEUTICAL SCIENCES, Chapter 21, 16th Edition (1980) for a general discussion of suitable types of emulsifiers and their uses. If present, the amount of emulsifier employed in compositions used in the method of this invention generally will be in the range of about 0.005 to about 5.0 percent by weight, more preferably in the range of about 0.05 to about 0.2 percent by weight of the formulation.

A dispersion medium also will be present in the intranasal composition used in this invention. The medium may be composed of a variety of substances such as water and other similar substantially non-toxic materials. Preferably, however, the medium is composed of one or more substances also capable of acting as a propellant for the system. Examples of substances capable of being used are, for example, the Freons from E.I. DuPont de Nemours and Company such as Freon 114 (1,2-dichloro-1,1,2,2-tetrafluoroethane), Freon 12 (dichlorodifluoromethane) and Freon 11 (trichlorofluoromethane). The substances preferably are used in combination. While a variety of ingredients in assorted concentrations may be employed in the combinations, preferred combinations include about a 1:1 mixture of Freon 11 and Freon 12 by weight and about a 5:4:1 mixture of Freons 11:12:114 by weight. The amount of ingredients comprising the dispersion medium will be from about 80.0 to greater than about 99.9 percent by weight, more preferably from about 95.0 to greater than about 99.9 percent by weight of the total formulation.

Due to the nasal route of administration, a scenting agent may be added also to the composition, if desired. When used, the scenting agent will be present in the range of about 0.001 to about 1.0 percent by weight. Examples of scenting agents are, for example, peppermint oil, lemon oil, olive oil and other like substances having a soothing effect on the olfactory system.

Certain of the previously mentioned emulsifiers unexpectedly are capable of enhancing intranasal absorption of insulin when present in the insulin formulation. A further aspect of this invention, therefore, comprises adding to the insulin formulation an absorption enhancing amount of (1) oleic acid or an ester or salt thereof, (2) a liquid form sorbitan fatty acid ester, (3) a liquid form polyoxyethylene derivative of a sorbitan fatty acid ester, or (4) a liquid form hydroxypolyoxyethylene-polyoxypropylene-polyoxyethylene co-polymer.

Of the first class of absorption enhancing agents, typical esters are methyl oleate, ethyl oleate, and the like, and typical salts are alkali metal salts such as sodium oleate, potassium oleate, and the like. Of the foregoing, oleic acid and ethyl oleate and preferred, oleic acid being most preferred.

Typical examples of liquid form sorbitan fatty acid ester absorption enhancing agents are those available commercially under the trademark Span. Specific examples are Span 20, Span 80, Span 85, and the like. The most preferred such agent is Span 85.

Typical examples of liquid form polyoxyethylene derivatives of sorbitan fatty acid ester absorption enhancing agents are those available commercially under the trademark Tween. Specific examples are Tween 20, Tween 21, Tween 40, Tween 60, Tween 80, Tween 85, and the like. The most preferred such agent is Tween 85.

Typical examples of liquid form hydroxypolyoxyethylene-polyoxypropylene-polyoxyethylene co-polymer absorption enhancing agents are those available commercially under the trademark Pluronic. Specific examples are Pluronic L-31, Pluronic L-35, Pluronic L-42, Pluronic L-43, Pluronic L-44, Pluronic L-61, Pluronic L-62, Pluronic L-63, Pluronic L-64, Pluronic L-72, Pluronic L-81, Pluronic L-92, Pluronic L-101, Pluronic L-121, Pluronic L-122, and the like.

Of the foregoing classes of absorption enhancing agents, those of the classes represented by oleic acid and liquid form polyoxyethylene derivatives of sorbitan fatty acid esters are most preferred.

When using an absorption enhancing agent from any of these classes, the agent will be added to the insulin formulation in an amount to achieve enhancement of intranasal absorption of insulin. Typically, the amount of agent will range, on a weight basis, from about 0.25% to about 1% based upon the total final formulation. The preferred amount generally will be somewhat less and will range from about 0.25% to about 0.75% by weight based upon the final formulation.

Even more preferred formulations will contain an agent from the oleic acid class in combination with an agent from any of the other three classes. The amount of each agent will be that which is sufficient to achieve enhancement of intranasal absorption of insulin. The amounts present can be in any of a wide range; typically, however, each agent will be present in a range on a weight basis of from about 0.25% to about 1% based upon the final formulation. Preferably, the range will be narrower, each of the agents being present in the range of about 0.25% by weight to about 0.75% by weight. When in this latter range, highly preferred formulations are those having equivalent weight amounts of each of the agents.

When the formulations are composed of two absorption enhancing agents, preferably they are oleic acid (including its esters and salts) and a liquid form polyoxyethylene derivative of a sorbitan fatty acid ester. Typical highly preferred formulations are those in which the agents are oleic acid and Tween 85. Most preferably, each agent will be present in an amount ranging from about 0.4% by weight to about 0.6% by weight, and especially being present each in an equivalent amount. A highly useful such formulation is one containing about 0.5% by weight oleic acid and about 0.5% by weight Tween 85.

Each of the formulations containing one or more absorption enhancing agents generally will be formulated such that the final resulting pH is within the range of from about 6 to about 8.

Formulations of the present invention may be prepared by procedures well known to those skilled in the art of formulation chemistry. When the formulation contains a propellant and is in the form of a suspension, it preferably is prepared as follows. Prior to formulating, the insulin may be micronized with any of several devices employed in the art, such as a mechanical micronizer. Next, any emulsifying agent, scenting agent, preservative, and/or other component is combined with the insulin in a suitable cannister or container. The cannister may be metal or glass, is typical of those employed in the art for this purpose, and is capable of being pressurized. The cannister then is charged with one or more propellants as described previously while cooling the cannister to a temperature in the range of from about 5°C to about -50°C, and, more preferably, from about 0°C to about -20°C. The cannister then is sealed, typically with an aerosol type valve, and warmed to check for leakage. The formulation thus prepared is then suitable for administration to a warm-blooded animal in need of treatment for diabetes mellitus.

Physicians will determine the particular dosage of the insulin to be administered intranasally. The selected dosages will vary depending upon a number of consideratons including, for example, the severity of the diabetes mellitus condition, the particular characteristics of the patient receiving treatment, and the like.

An aqueous formulation preferably is prepared as follows. A solution containing the appropriate amount of any selected preservative, buffer, emulsifier, scenting agent, and/or other component is prepared. A measured amount of insulin is added to the solution, and sufficient vehicle is added to achieve the desired final concentration.

Particularly preferred intranasal compositions provided by this invention are as follows:

Solution Formulations

Formula 1

| Ingredient | Concentration |
| --- | --- |
| sodium human insulin | 100 Units (about 0.04% by weight) |
| phosphate buffered saline | q.s. (quantum sufficit) to 1 ml |

Formula 2

| Ingredient | Concentration |
|---|---|
| potassium porcine insulin | 100 Units (about 0.04% by weight) |
| benzalkonium chloride | about 0.01% to about 0.5% by weight |
| distilled water | q.s. to 1 ml |

Formula 3

| Ingredient | Concentration |
|---|---|
| free acid human insulin | 100 Units (about 0.04% by weight) |
| benzalkonium chloride | about 0.01% to about 0.3% by weight |
| EDTA | about 0.01% to about 0.3% by weight |
| phosphate buffered saline | q.s. to 1 ml. |

Formula 4

| Ingredient | Concentration |
|---|---|
| lithium human insulin | 200 Units (about 0.08% by weight) |
| benzalkonium chloride | about 0.1% by weight |
| distilled water | q.s. to 1 ml |

Formula 5

| Ingredient | Concentration |
|---|---|
| sodium human insulin | 200 Units (about 0.08% by weight) |
| methyl p-hydroxybenzoate | about 0.2% by weight |
| distilled water | q.s. to 1 ml |

Formula 6

| Ingredient | Concentration |
|---|---|
| sodium human insulin | 100 Units (about 0.04% by weight) |
| methyl p-hydroxybenzoate | about 0.1% by weight |
| propyl p-hydroxybenzoate | about 0.05% by weight |
| phosphate buffered saline | q.s. to 1 ml |

## Suspension Formulations

Formula 7

| Ingredient | Concentration |
|---|---|
| potassium bovine insulin | 100 Units (about 0.04% by weight) |
| Span 85 | about 0.05% by wight |
| Freon 12 | about 59.95% by weight |
| Freon 114 | about 39.96% by weight |

Formula 8

| Ingredient | Concentration |
|---|---|
| sodium human insulin | 200 Units (about 0.08% by weight |
| Span 85 | about 0.02% by weight |
| oleic acid | about 0.05% by weight |
| Freon 11 | about 49.93% by weight |
| Freon 12 | about 49.92% by weight |

**0 200 383**

Formula 9

| Ingredient | Concentration |
|---|---|
| potassium human insulin | 100 Units (about 0.04% by weight |
| Tween 85 | about 0.05% by weight |
| Span 85 | about 0.01% by weight |
| oleic acid | about 0.05% by weight |
| methyl p-hydroxybenzoate | about 0.2% by weight |
| Freon 11 | about 39.86% by weight |
| Freon 12 | about 59.79% by weight |

Formula 10

| Ingredient | Concentration |
|---|---|
| sodium human insulin | 50 Units (about 0.02% by weight) |
| Span 85 | about 0.02% by weight |
| lecithin | about 0.05% by weight |
| Freon 11 | about 39.96% by weight |
| Freon 12 | about 34.97% by weight |
| Freon 114 | about 24.98% by weight |

Formula 11

| Ingredient | Concentration |
|---|---|
| sodium human insulin | 0.5% by weight |
| oleic acid | 0.5% by weight |
| Tween 85 | 0.5% by weight |
| Freon 11 | 49.25% by weight |
| Freon 12 | 24.625% by weight |
| Freon 114 | 24.625% by weight |

Formula 12

| Ingredient | Concentration |
|---|---|
| sodium human insulin | 1.0% by weight |
| oleic acid | 0.5% by weight |
| Tween 85 | 0.5% by weight |
| Freon 11 | 49.0% by weight |
| Freon 12 | 24.5% by weight |
| Freon 114 | 24.5% by weight |

Formula 13

| Ingredient | Concentration |
|---|---|
| sodium human insulin | 2.0% by weight |
| oleic acid | 0.5% by weight |
| Tween 85 | 0.5% by weight |
| Freon 11 | 48.5% by weight |
| Freon 12 | 24.25% by weight |
| Freon 114 | 24.25% by weight |

The following non-limiting examples illustrate specific intranasal compositions used in this invention. Also compared is their effectiveness to corresponding zinc insulin compositions.

Insulin formulations for Examples 1 to 3 were prepared using the following insulins: zinc porcine insulin, sodium porcine insulin, zinc human insulin, sodium human insulin, and metal-free human insulin. The sodium and metal-free insulins were dissolved in distilled deionized water. For the zinc insulin solutions, 0.05 $\underline{M}$ $KH_2PO_4$ buffer (pH 7.4) or distilled deionized water (adjusted with HCl and NaOH to pH 7.4) was used as solvent. All solutions were prepared to contain about 10 to about 13 Units of insulin per milliliter.

The formulations were tested in non-fasted male Sprague-Dawley rats (200-300 gm). On the day of the experiment the rats were transferred to a surgical table where they could comfortably lie on their back. A surgical procedure was followed as described in Hussain, A., Hirai, S., and Banarshi, R., $\underline{J}$. $\underline{Pharm}$. $\underline{Sci}$. 69, 1411-1413 (1980); and Su, K.S.E., Campanale, K.M., and Gries C.L., $\underline{J}$. $\underline{Pharm}$. $\underline{Sci}$. 73, 1251-1254 (1984). The procedure is summarized as follows. The rats were anesthetized with 50 mg/kg dose of pentobarbital by intraperitoneal injection 30 minutes prior to surgery. An incision was made in the neck, and the trachea was cannulated with a polyethylene tube.

Another tube was inserted from the esophagus to the posterior part of the nasal cavity. The nasopalatine was closed with an adhesive agent to prevent drainage of the drug solution from the nasal cavity to the mouth. The drug solution was delivered to the nasal cavity through the esophagus cannulation tubing by means of a syringe. Twenty microliters of blood were sampled from the tail vein periodically. The blood sample was immediately mixed in a sample cup with 480 $\mu$l of an anticoagu- lant solution (prepared by dissolving 2.0 gm of disodium ethylenediaminetetraacetic acid dihydrate and 0.4 gm of sodium fluoride in deionized water q.s. to 2.0 liters) and assayed by the glucose oxidase-peroxidase method for the measurement of glucose in serum or plasma. This method, as modi- fied by P. Trinder [$\underline{Ann}$. $\underline{Clin}$. $\underline{Biochem}$., 6, 24 - (1969), and $\underline{J}$. $\underline{Clin}$. $\underline{Pathol}$., 22, 246 (1969)] involves two reactions to determine glucose concentrations.

First, the oxidation of glucose in the presence of glucose oxidase yields gluconic acid and peroxide. The peroxide then reacts with phenol and 4-aminoantipyrine in the presence of peroxidase to yield a red dye measureable at 500-520 nm. The method is designed for use in automated and semi-automated equipment.

The protocol for collecting blood samples was designed as follows. A blood sample was collected from the tail vein at 5, 3, and 1 minute prior to administration of insulin and at 1, 5, 10, 20, 30, 40, 60, 90, 120, 150 and 180 minutes following administration of insulin. Each sample was treated as described above.

The profile of changes in blood glucose level following administration of insulin was determined as follows. The blood glucose level immediately prior to insulin administration is represented as 100%. The blood glucose level at time t is expressed as percent of the pre-injection blood glucose using the equation

$$\frac{Ct}{Co} \times 100\%$$

in which Ct is blood glucose concentration at time t and Co is blood glucose concentration immediately prior to insulin administration. For the purpose of relative comparison, the decrement of the blood glucose level was integrated using the area under the change in blood glucose versus time curve - (AUC) from 0 to 180 minutes, which corresponds to the area above the blood glucose concentration curve but under the 100% level. The AUC was calculated by the trapezoidal method using a computer.

Example 1 --Comparison of Blood Glucose Lowering Effect from Intranasal Administration of Zinc Porcine Insulin and Sodium Porcine Insulin

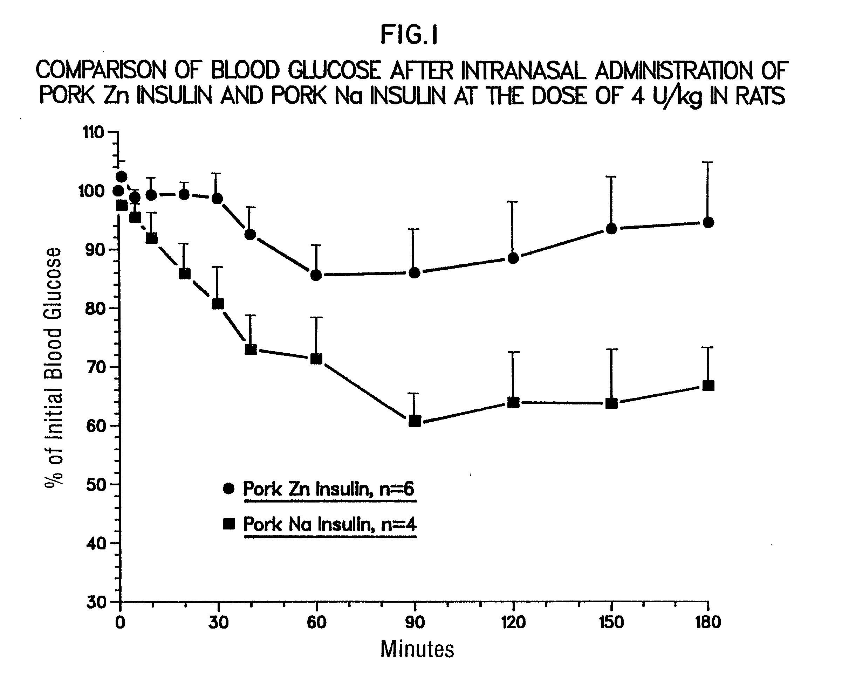

The change in blood glucose levels following intranasal administration of 4 units/kg of the zinc porcine insulin is shown in Figure 1. The zinc porcine insulin produces slight hypoglycemic activity. The decline in blood glucose began at about 40 minutes following intranasal administration and the nadir of blood glucose decline reached about 16% at 60 minutes and then gradually recovered to the base line. The results are consistent with those of previous reports which suggest that little or no blood glucose depression is observed following intranasal administration of zinc insulin in rats, dogs, and humans. However, when the same dose of sodium porcine insulin (4 units/kg) was intranasally administered, the blood glucose concentration fell promptly after 5 to 10 minutes as shown in Figure 1. The nadir of blood glucose occurred at about 90 minutes, with depression of about 40% or more after which gradual recovery was observed. Comparison of blood glucose lowering effect after intranasal administration of zinc porcine insulin and sodium porcine insulin in rats is summarized in Table 1 following. The relative absorption following intranasal administration when compared to subcutaneous administration is about 23% in the case of zinc porcine insulin and 66% in that of sodium porcine insulin. Moreover, the statistical significance of the difference of intranasal absorption between zinc porcine insulin and sodium porcine insulin is $p < 0.05$.

## Table 1

### Comparison of Blood Glucose Level Following Intranasal Administration of Zinc Porcine Insulin and Sodium Porcine Insulin in Rats[a]

| Sample[b] | Administration Route | Dose (unit/kg) | No. of Animals | AUC[c] | Mean Sp[d] AUC | $AUC_{ns}/AUC_{sc}$[f] |
|---|---|---|---|---|---|---|
| Zinc Insulin | Subcu. | 5 | 3 | 11424 (±440) | 2285 | --- |
| Zinc Insulin | Nasal | 4 | 6 | 2052 (±932) | 513 | 22.4% |
| Sodium Insulin | Subcu. | 5 | 4 | 10267 (±460) | 2053 | --- |
| Sodium Insulin | Nasal | 4 | 4 | 5402[e] (±1053) | 1350 | 65.8% |

[a] Male Sprague-Dawley, non-fasted.

[b] Aqueous solution.

[c] 0-3 hours, percent change in blood glucose x minutes.

[d] Mean specific AUC = mean AUC/administered dose.

[e] $p < 0.05$, compared to zinc insulin by nasal route.

[f] Intranasal mean specific AUC/Subcutaneous mean specific AUC x 100.

Example 2 --Comparison of Blood Glucose Lowering Effect from Intranasal Administration of Zinc Human Insulin and Sodium Human Insulin

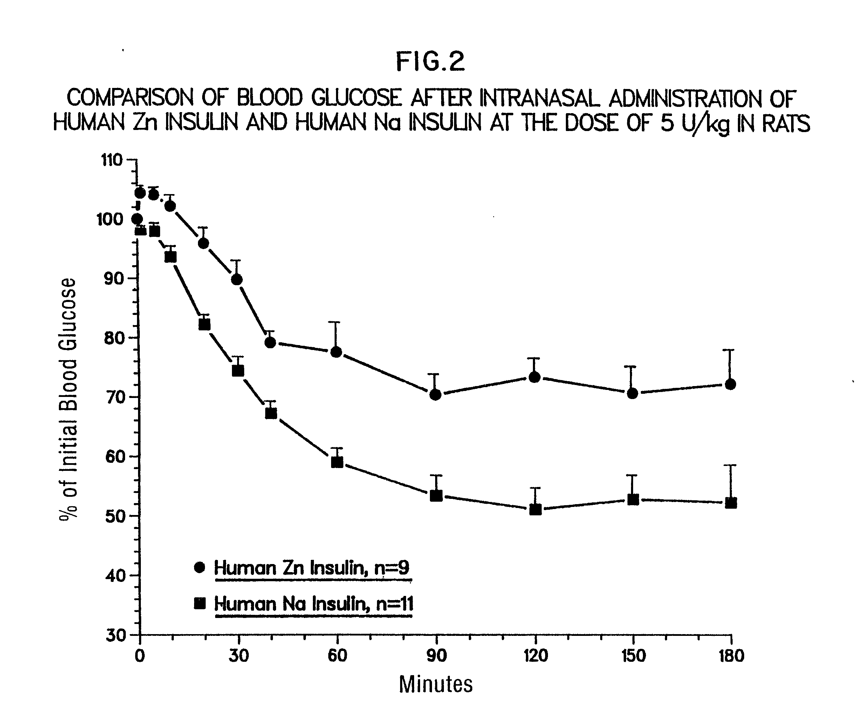

The change in blood glucose levels following intranasal administration of 5 units/kg of zinc human insulin is shown in Figure 2. In a pattern similar to zinc porcine insulin, the zinc human insulin produced slight blood glucose lowering effect. The decline in blood glucose began about 20 minutes following intranasal administration. The peak blood glucose decline reached about 70% of initial at 90 minutes and then gradually recovered toward the base line; whereas, the peak decline in blood glucose by sodium insulin was approximately 50%. Comparison of blood glucose lowering effect following intranasal administration of zinc human insulin and sodium human insulin in rats is summarized in Table 2. It is apparent that an increased response is available following intranasal administration.

istration of sodium human insulin. Moreover, the statistical significance of the difference of blood glucose lowering effect between zinc human insulin and human sodium insulin is p <0.001.

## Table 2

Comparison of Blood Glucose Level Following Intranasal Administration of Zinc Human Insulin and Sodium Human Insulin in Rats[a]

| Sample[b] | Administration Route | Dose (unit/kg) | No. of Animals | AUC[c] | $\frac{AUC_{ns}}{AUC_{sc}}$ [e] |
|---|---|---|---|---|---|
| Zinc Insulin | Subcu. | 5 | 4 | 10481 (±672) | --- |
| Zinc Insulin | Nasal | 5 | 9 | 4064 (±422) | 38.8% |
| Sodium Insulin | Subcu. | 5 | 3 | 10757 (±741) | --- |
| Sodium Insulin | Nasal | 5 | 11 | 7063[d] (±507) | 65.7% |

[a] Male Sprague-Dawley, non-fasted.
[b] Aqueous solution.
[c] 0-3 hours, percent change in blood glucose x minutes.
[d] p <0.001, compared to zinc insulin by nasal route.
[e] Intranasal AUC/subcutaneous AUC x 100.

Example 3 -- Comparison of Blood Glucose Lowering Effect from Intranasal Administration of Metal-Free Human Insulin and Sodium Human Insulin

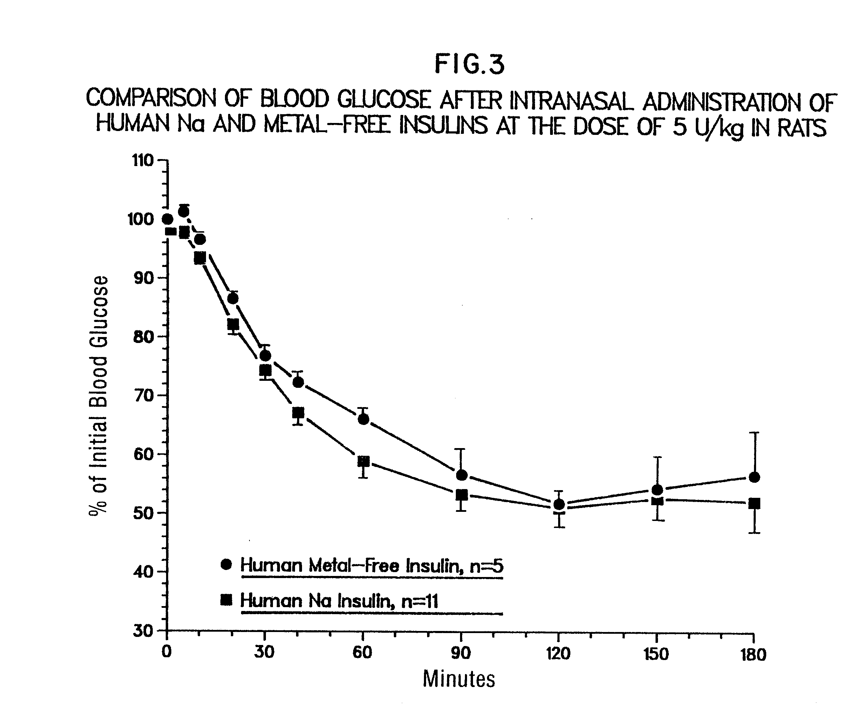

The change in blood glucose levels following intranasal administration of 5 units/kg of metal-free human insulin is shown in Figure 3. In a pattern similar to sodium human insulin, the metal-free human insulin produced a substantial decline in blood glucose. Comparison of blood glucose lowering effect following intranasal administration of sodium human insulin and metal-free human insulin in rats is summarized in Table 3. It is apparent that the blood glucose lowering response is about the same for both sodium human insulin and metal-free human insulin administered intranasally.

Table 3

Comparison of Blood Glucose Level Following Intranasal Administration of Sodium Human Insulin and Metal-Free Human Insulin in Rats[a]

| Sample[b] | Administration Route | Dose (unit/kg) | No. of Animals | AUC[c] |
|---|---|---|---|---|
| Sodium Insulin | Nasal | 5 | 11 | 7063 (±507) |
| Metal-Free Insulin | Nasal | 5 | 5 | 6407 (±726) |

[a] Male Sprague-Dawley, non-fasted.
[b] Aqueous solution.
[c] 0-3 hours, percent change in blood glucose x minutes.

Example 4 --Intranasal Administration of Sodium Human Insulin Formulations Containing Absorption Enhancing Agents

Employing the previously described methods, formulations of sodium human insulin containing one or more absorption enhancing agents were administered intranasally to dogs. The formulations were prepared by fitting an appropriate container with a mechanical stirrer and a cooling bath. The desired amounts of absorption enhancing agent(s), sodium human insulin, and Freon 11 were mixed in the container until uniform. An appropriate amount of the resulting insulin suspension concentrate then was weighed into an aerosol canister. A valve was added and sealed onto the canister. Desired amounts of Freon 12 and Freon 114 propellants then were added using a pressure-filling method.

Three parameters, insulin peak, AUC, and glucose level at nadir, were measured. These results, in their composite, show the enhancement of absorption by the presence of one or more of the absorption enhancing agents when compared to formulations lacking such agents. Table 4 following provides the results from these studies.

## Table 4

### Effect of Oleic Acid and/or Tween 85 on Nasal Absorption of Human Sodium Insulin in Dogs

| Insulin U/kg | Oleic Acid (% w/w) | Tween 85 (% w/w) | No. of Dogs | Insulin Peak Level (μU/mL) | Insulin Peak Time (min) | AUC$\int_0^{240}$ (μU·min·mL$^{-1}$) | Glucose Level at Nadir (% of Initial) | Nadir Time (min) |
|---|---|---|---|---|---|---|---|---|
| 0 | 0.5 | 0.5 | 4 | 13.5 ± 7.4 | - | 2274 ± 291 | 95.4 ± 3.5 | - |
| 0[a] | 0 | 0 | 4 | 7.6 ± 2.6 | - | 1264 ± 95 | 94.8 ± 0.8 | - |
| 0.8 | 0 | 0.1 | 4 | 29.2 ± 2.8 | 10 | 3598 ± 413 | 83.5 ± 2.8 | 45 |
| 0.8 | 0 | 0 | 4 | 23.3 ± 4.7 | 10 | 3166 ± 398 | 79.6 ± 12.0 | 60 |
| 0.8 | 0 | 1.0 | 4 | 40.5 ± 6.0 | 10 | 4193 ± 468 | 71.3 ± 5.9 | 45 |
| 0.8 | 0.25 | 0.1 | 4 | 49.2 ± 2.4 | 10 | 4327 ± 206 | 69.4 ± 3.8 | 60 |
| 0.8 | 0 | 0.5 | 4 | 38.6 ± 3.7 | 10 | 4227 ± 339 | 68.5 ± 7.4 | 60 |
| 0.8 | 0.5 | 0.25 | 4 | 41.9 ± 4.5 | 10 | 4316 ± 323 | 67.2 ± 8.5 | 45 |
| 0.8 | 0.5 | 0.1 | 7 | 31.2 ± 2.8 | 10 | 3778 ± 433 | 65.9 ± 6.9 | 60 |
| 0.8 | 0.5 | 0 | 4 | 44.3 ± 6.1 | 10 | 3687 ± 468 | 64.8 ± 2.0 | 45 |
| 0.8 | 1.0 | 1.0 | 4 | 46.5 ± 11.5 | 10 | 3773 ± 722 | 64.1 ± 12.2 | 45 |
| 0.8 | 1.0 | 0.1 | 4 | 51.4 ± 6.1 | 20 | 4337 ± 533 | 59.8 ± 3.9 | 60 |
| 0.8 | 1.0 | 0.1 | 8 | 37.9 ± 4.6 | 10 | 3597 ± 494 | 59.6 ± 4.9 | 60 |

Table 4 continued

| Insulin U/kg | Oleic Acid (% w/w) | Tween 85 (% w/w) | No. of Dogs | Insulin Peak Level (μU/mL) | Insulin Peak Time (min) | AUC$\int_0^{240}$ (μU·min·mL$^{-1}$) | Glucose Level at Nadir (% of Initial) | Nadir Time (min) |
|---|---|---|---|---|---|---|---|---|
| 0.8 | 0.25 | 0.25 | 4 | 64.1 ± 6.6 | 10 | 3933 ± 268 | 58.3 ± 8.9 | 45 |
| 0.8 | 0.75 | 0.1 | 4 | 37.7 ± 0.8 | 10 | 3546 ± 361 | 54.7 ± 5.0 | 60 |
| 0.8 | 0.25 | 0.5 | 3 | 55.7 ± 11.0 | 10 | 3463 ± 558 | 51.6 ± 5.7 | 45 |
| 0.8 | 0.5 | 0.5 | 14 | 80.2 ± 8.6 | 10 | 5428 ± 461 | 47.4 ± 3.6 | 45 |
| 0.8 | 0.5[b] | 0 | 4 | 36.6 ± 6.9 | 10 | 4083 ± 505 | 62.6 ± 8.0 | 30 |
| 0.8 | 0 | 0.5[c] | 4 | 42.8 ± 4.0 | 10 | 3209 ± 211 | 69.7 ± 9.1 | 45 |
| 0.8 | 0 | 0.5[d] | 4 | 41.7 ± 2.6 | 10 | 3919 ± 330 | 81.4 ± 4.0 | 45 |
| 0.8 | 0 | 0.5[e] | 4 | 58.6 ± 13.1 | 20 | 8714 ± 1349 | 66.7 ± 7.8 | 90 |

[a]Aqueous solution of phosphate buffer.

[b]Ethyl oleate.

[c]Span 85.

[d]Tween 80.

[e]Pluronic L-101.

## Claims

1. An intranasal pharmaceutical formulation containing insulin for the treatment of diabetes mellitus characterized in that said intranasal formulation is comprised of insulin as an alkali metal salt, the ammonium salt or the free acid of a substantially zinc-free insulin, and a pharmaceutically acceptable diluent therefor.

2. An intranasal pharmaceutical formulation as claimed in claim 1 characterized further in that it contains an absorption enhancing amount of at least one absorption enhancing agent selected from the group consisting of (1) oleic acid or an ester or salt thereof, (2) a liquid form sorbitan fatty acid ester, (3) a liquid form polyoxyethylene derivative of a sorbitan fatty acid ester, and (4) a liquid form hydroxypolyoxyethylene-polyoxypropylene-polyoxyethylene copolymer.

3. A formulation as claimed in claim 2 in which the absorption enhancing agent is oleic acid or an ester or salt thereof.

4. A formulation as claimed in claim 3 in which oleic acid is present in an amount of from about .25% to about 1% by weight of the final formulation.

5. A formulation as claimed in claim 2 in which the absorption enhancing agent is a liquid form polyoxyethylene derivative of a sorbitan fatty acid ester.

6. An intranasal formulation as claimed in claim 5 in which the absorption enhancing agent is Tween 85 present in an amount of from about .25% to about 1% by weight of the final formulation.

7. An intranasal formulation as claimed in claim 2 in which the absorption enhancing agent is present as a combination of (1) oleic acid or an ester or salt thereof and (2) a liquid form polyoxyethylene derivative of a sorbitan fatty acid ester.

8. An intranasal formulation as claimed in claim 7 in which the absorption enhancing agent is oleic acid and Tween 85 each present in an amount of from about .25% to about 1% by weight of the final formulation.

9. An intranasal pharmaceutical formulation as claimed in any one of claims 1 to 8 in which the insulin is sodium insulin.

10. An intranasal pharmaceutical formulation as claimed in claim 9 in which the insulin is human insulin.

11. An intranasal pharmaceutical formulation which comprises oleic acid and Tween 85, each of which are present in an amount of about .5% by weight based upon the final formulation, and human sodium insulin.

12. The ammonium salt, an alkali metal salt, or the free acid of a substantially zinc-free insulin for use in the intranasal treatment of diabetes mellitus.

13. The sodium salt of an insulin for use in the intranasal treatment of diabetes mellitus.

14. Human insulin as its sodium salt for use in the intranasal treatment of diabetes mellitus.

15. An intranasal formulation as claimed in any one of claims 1 to 11 for use in the intranasal treatment of diabetes mellitus.

16. The use of the ammonium salt, an alkali metal salt, or the free acid of a substantially zinc-free insulin for the manufacture of a medicament for the intranasal treatment of diabetes mellitus.

17. The use claimed in claim 16 in which the insulin is in the form of its sodium salt.

18. The use claimed in claim 17 in which the insulin is human insulin.

## FIG.1

COMPARISON OF BLOOD GLUCOSE AFTER INTRANASAL ADMINISTRATION OF
PORK Zn INSULIN AND PORK Na INSULIN AT THE DOSE OF 4 U/kg IN RATS

● Pork Zn Insulin, n=6
■ Pork Na Insulin, n=4

# FIG.2

COMPARISON OF BLOOD GLUCOSE AFTER INTRANASAL ADMINISTRATION OF
HUMAN Zn INSULIN AND HUMAN Na INSULIN AT THE DOSE OF 5 U/kg IN RATS

Legend:
- ● Human Zn Insulin, n=9
- ■ Human Na Insulin, n=11

Y-axis: % of Initial Blood Glucose
X-axis: Minutes

# FIG.3

COMPARISON OF BLOOD GLUCOSE AFTER INTRANASAL ADMINISTRATION OF HUMAN Na AND METAL–FREE INSULINS AT THE DOSE OF 5 U/kg IN RATS

● Human Metal–Free Insulin, n=5

■ Human Na Insulin, n=11

0 200 383